# EUROPEAN PATENT APPLICATION

(11) **EP 3 470 069 A1**
(43) Date of publication of application: **17.04.2019**
(21) Application number: 18461563.1
(22) Date of filing: 30.05.2018
(51) Int. Cl.: A61K 31/222, A61P 9/10

(54) **OLEACEIN FOR USE IN ATHEROSCLEROSIS PREVENTION**

(30) Priority: 12.10.2017 PL 42314617
(71) Applicant: Warszawski Uniwersytet Medyczny, 02-091 Warszawa (PL)
(72) Inventor: Filipek, Agnieszka, 05-807 Zolwin/Podkowa Lesna (PL); Naruszewicz, Marek, 05-540 Zalesie Górne (PL)
(74) Representative: Witek, Rafal

(57) **Abstract**

Disclosed is the use of oleacein to inhibit foam cell formation process by effective inhibition of oxLDL uptake by macrophages, particularly by reducing expression of macrophage scavenger receptors. Oleacein-comprising pharmaceutical preparations produced according to the invention are viable for use in the treatment and/or prevention of atherosclerosis, particularly in the prevention of early atherosclerotic lesions.

## Description

The object of the invention is the use of oleacein to inhibit foam cell formation process by effective inhibition of oxLDL uptake by macrophages, particularly as a result of reduced expression of macrophage scavenger receptors. Oleacein-comprising pharmaceutical preparations produced according to the invention are viable for use in the treatment and/or prevention of atherosclerosis, particularly in the prevention of early atherosclerotic lesions.

Oleacein is a compound of formula 1, in the literature also referring to as 3,4-DHPEA-EDA, which is 3,4-dihydroxyphenylethanol (hydroxytyrosol) esterified with dialdehydic derivative of elenoic acid.

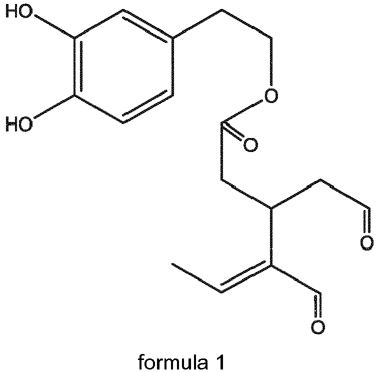

Czerwińska M. et al., EAS 2012, abstract book 2012-05-29, p. 1108, disclosed the results of the study on the effect of oleacein on myeloperoxidase release (MPO), carried out using selected group of cells, i.e. neutrophils isolated from peripheral blood of healthy volunteers *in vitro.* Additionally, the effect of oleacein on the release of MPO from blood-isolated neutrophils is known from Czerwińska M. et al., Food Chemistry, vol. 131, no. 3. 2011-09-20, p. 940-947. The results indicate protective effect of oleacein against oxidative damages.

Atherosclerosis is a syndrome based on endothelium dysfunction and chronic inflammation. Monocytes, macrophages and modified low-density lipoproteins (LDL), mostly oxidized fraction (oxLDL), are believed to play the main role in atherosclerosis pathogenesis. Damage of endothelium cells (EC) by oxLDL initiates a series of proinflammatory reactions, which result in activation of adhesive molecules (ICAM-1, VCAM-1) and chemoattractants (M- CSF, MCP-1). This leads to an increased migration of monocytes towards the inner membrane of blood vessels. At that location, monocytes transform into macrophages, which uptake oxLDL and transform into foam cells. Foam cells are the primary structural component of atherosclerotic plaques and play an important role in the development of atherosclerosis, which leads to a stroke and/or myocardial infarction and death.

oxLDL binding by macrophage cells precedes the uptake process and involves scavenger receptors (SRs). Scavenger receptors are proteins and they play an important role in physiological and pathological processes of the whole human body. Their function is to bind modified lipoproteins, polyanions and cells in apoptosis or necrosis phase. There are 7 classes of SRs, from SR-A to SR-G. In the accumulation of lipid deposits (foam cells), mainly receptors from A class - SRA (CD204), from B class - CD36 and from E class - LOX-1 (lectin-like oxLDL receptor) are engaged.

Polish patent application P.399962 discloses the use of *Ligustrum vulgare L.* for manufacturing a preparation comprising oleacein for the inhibition of MMP-9 expression by the cells present in atherosclerotic plaques and for atherosclerotic plaque stabilisation, particularly for the treatment and prevention of diseases resulting from a disintegration of atherosclerotic plaque, especially those selected from the group comprising: ischaemic stroke, myocardial diffraction and ischaemic heart disease.

The aim of the invention is to provide a preparation which could be used to inhibit foam cell formation process by effective inhibition of oxLDL uptake by macrophages, particularly resulted from reduced expression of macrophage scavenger receptors. Such preparation could be used for the treatment and prevention of atherosclerosis, particularly for the prevention of early atherosclerosis stages.

Surprisingly, the aim as set forth herein is realised in the subject invention.

The object of the present invention is an oleacein for the use in in-vivo inhibition of foam cell formation process and/or in the inhibition of the formation of lipid deposits in macrophages, preferably by an effective inhibition of oxLDL uptake by macrophages, particularly as a result of reduced expression of scavenger receptors on macrophages. Preferably, oleacein reduces expression of scavenger receptor selected from: CD36, LOX-1 and SRA (CD204). Preferably, oleacein is intended for use in the treatment or prevention of atherosclerosis, particularly in the prevention of early atherosclerotic stages.

Preferably, oleacein derived from common privet *(Ligustrum vulgare L.)* is used, particularly oleacein isolated by the method disclosed in patent application P.399962.

Potential contribution of oleacein into the stabilisation of human atherosclerotic plaque has been shown already (patent application P.399962). The present invention proves that oleacein can be used also in the prevention of early atherosclerotic lesions by the inhibition of processes preceding atherosclerotic plaque formation.

### Example 1. Preparation of oleacein from leaves of common privet (Ligustrum vulgare L.)

Oleacein can be obtained from various plants from Oleacea family, i.e. olive tree (Olea *europea L*.) and common privet *(Ligustrum vulgare L.),* particularly from the leaves of common privet. Common privet *(Ligustrum vulgare L.)* is an ornamental plant common in Europe, which is often used for planting hedges. Oleacein from the leaves of common privet was isolated using the method disclosed in patent application P.399962.

400 g of grounded raw material was used for the isolation. In the first stage, the leaves of common privet were extracted four times with distilled water at 30°C using ultrasonic cleaner, each time for 30 minutes. Extractions were performed at raw material:solvent ratio of 1:10. Aqueous extracts were filtered through cotton wool and pooled. The obtained aqueous extract was concentrated by lyophilisation to the final volume of about 1L. In order to purify the extract further and to increase the efficiency of the desired compound isolation process, diethyl ether was used instead of ethyl acetate. As shown in previous analyses, extract prepared with ethyl acetate contains more chemical compounds and thus creates difficulties in oleacein isolation process. Concentrated aqueous extract was then subjected 5 times to extraction with diethyl ether in solvent:extract ratio of 1:1. Ether extract was concentrated on a rotavapour under reduced pressure at 35°C. About 5 g of ether extract was obtained. The ether extract was subjected to separation by flash chromatography on a silica gel column (PF- 30 SIHP/80G PuriFlash) using mixture of chloroform and ethyl acetate (85:15) in isocratic mode for 60 minutes (20 mL/min). A total of 9 fractions was obtained, and fraction 3 and 4 were selected for further separation. Those fractions were subjected to further separation on a silica gel column (PF- 30 SIHP/80G PuriFlash) using mixture of toluene, methyl acetate and methanol (84:11:5) in isocratic mode for 60 minutes (20 mL/min). A total of 5 fractions was obtained. Fraction 3 was loaded onto the Sephadex column and oleacein was isolated therefrom using mixture of chloroform and methanol (9:1). A total of 1.289 g of the compound was obtained. The identity of the compound was confirmed by NMR and HPLC-DAD-MS/MS methods.

### Example 2. Oleacein as a compound that inhibits formation of foam cells from human macrophages during atherosclerosis development.

All experiments were conducted with the use of macrophage cells isolated from human peripheral blood. Macrophages were incubated for 72 hours with oxLDL and with oleacein at concentrations of 20 µmol/L and 50 µmol/L. Oleacein doses used showed no cytotoxicity on macrophage cells. Expression of scavenger receptors was determined using flow cytometer. Visualization of lipid deposits in macrophages (foam cells) was carried out with the use of well-known Red Oil O staining method.

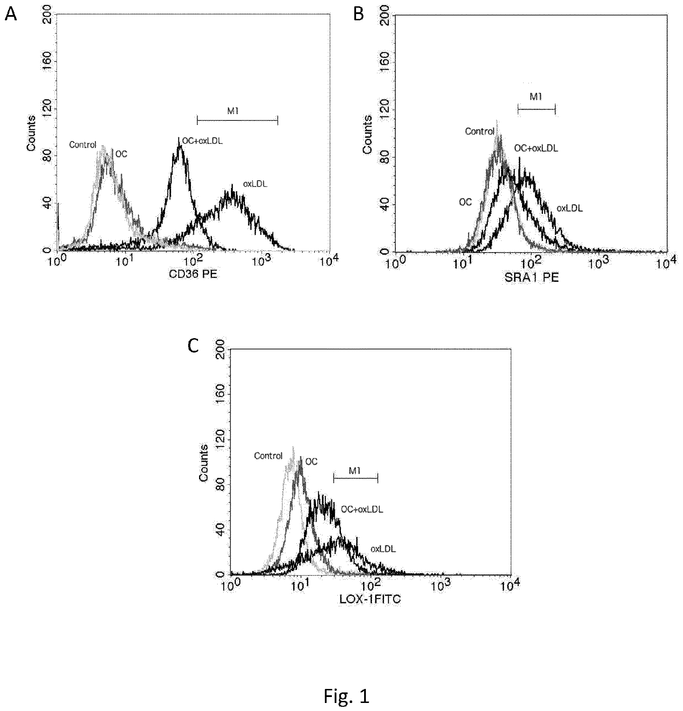

The results obtained are shown in Fig 1. The effect of oleacein on the expression of scavenger receptors, i.e. SRA (A), CD36 (B) and LOX-1 (C) in foam cells was determined with the use of flow cytometer and shown as histograms. In the histograms, the M1 marker shows maximum expression of SRA, CD36 and LOX-1 receptors under macrophage stimulation with oxLDL. Control - cells without stimulation, OC - the cells incubated with oleacein (50 µmol/L), OC+oxLDL - the cells incubation with oleacein (50 µmol/L) and oxLDL (50 µg/mL), oxLDL - the cells stimulated with oxLDL (50 µg/mL) - stimulated control.

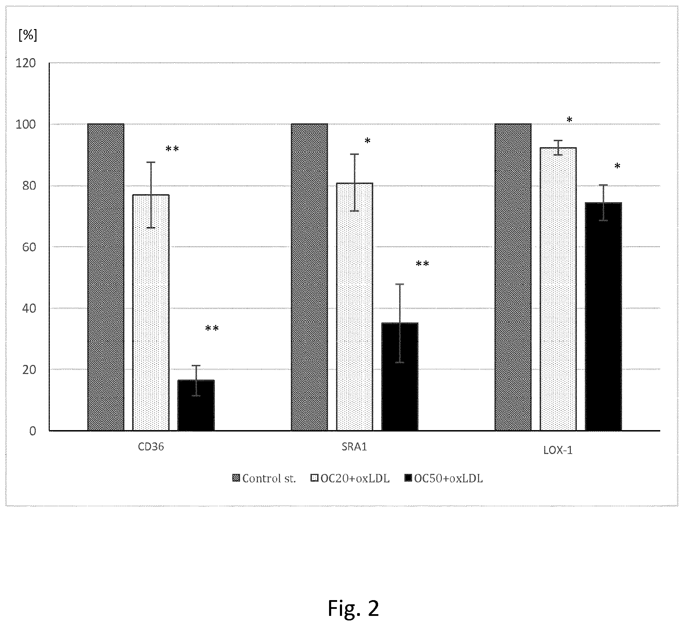

The results of experiments evaluating the effect of oleacein on the expression of scavenger receptors, i.e. CD36, SRA and LOX-1, in foam cells, are summarised in Fig 2. The following designations were used: # - statistical significance (* - p<0.05, ** - p<0.001) was determined for oxLDL-stimulated cells at the concentration of 50 µg/mL (stim. contr.). OC20+oxLDL - macrophage cells incubated with oleacein at 20 µmol/L and oxLDL at 50 µg/mL, OC50+oxLDL - macrophage cells incubated with oleacein at 50 µmol/L and oxLDL at 50 µg/mL.

In conclusion, the results obtained prove that oleacein significantly reduced expression of macrophage scavenger receptors. Demonstrated oleacein activity was dependent on the dose used. The largest decrease in receptor activity was reported for CD36 (from about 23% to about 84%, p<0.001), and the smallest decrease was noticed for LOX-1 (from about 8% to about 25%, p<0.05). On the other hand, about 20% and about 75% (p<0.05; p<0.001) decrease in receptor activity was reported for SRA.

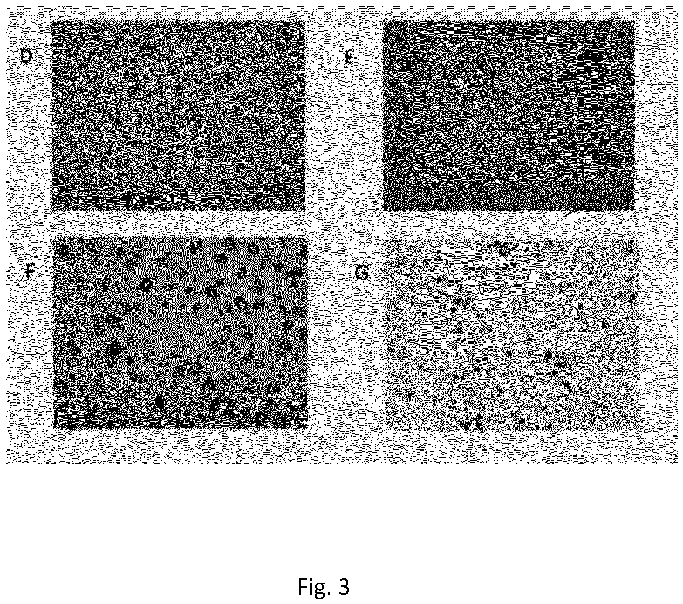

Moreover, visualisations of lipid deposits observed in macrophage foam cells with the use of Red Oil O, are shown in Fig. 3. The images were acquired using fluorescence microscope Nikon TS100F. D - control, the cells without stimulation, E - the cells incubated with oleacein (50 µmol/L), F - the cells stimulated with oxLDL (50 µg/ml) - stimulated control, G - the cells incubated with oleacein (50 µmol/L) and oxLDL (50 µg/mL).

So far, oleacein is the only natural substance shown to have an inhibitory effect on the formation of foam cells and lipid deposits.

## Claims

1. Oleacein for use in *in vivo* inhibition of foam cells formation process and/or in inhibition of lipid deposit formation in macrophage cells.

2. Oleacein for use according to claim 1, **characterised in that** it is intended to inhibit uptake of oxLDL by macrophages.

3. Oleacein for use according to claim 1, **characterised in that** it is intended to reduce the expression of macrophage scavenger receptors.

4. Oleacein for use according to claim 1, **characterised in that** it is intended to reduce the expression of macrophage scavenger receptor selected from: CD36, LOX-1 and SRA (CD204).

5. Oleacein for use according to claim 1, **characterised in that** it is intended for use in atherosclerosis treatment and/or prevention.

6. Oleacein for use according to claim 1, **characterised in that** it is intended for use in the prevention of early arteriosclerotic lesions.

7. Oleacein for use according to claim 1, **characterised in that** it is produced from common privet *(Ligustrum vulgare L.).*
